Europäisches Patentamt

European Patent Office

Office européen des brevets

(11) Numéro de publication: **0 421 853 A1**

# DEMANDE DE BREVET EUROPEEN

(21) Numéro de dépôt: 90402703.4

(51) Int. Cl.5: **C07C 55/02**, C07C 51/14

(22) Date de dépôt: **01.10.90**

(30) Priorité: **02.10.89 FR 8913055**

(43) Date de publication de la demande:
**10.04.91 Bulletin 91/15**

(84) Etats contractants désignés:
**AT BE CH DE DK ES FR GB GR IT LI LU NL SE**

(71) Demandeur: **RHONE-POULENC CHIMIE**
**25, quai Paul Doumer**
**F-92408 Courbevoie Cédex(FR)**

(72) Inventeur: **Brunet, Jean-Jacques**
**5, rue de la Résistance, Pinsaguel**
**F-31120 Portet Sur Garonne(FR)**
Inventeur: **Passelaigue, Elisabeth**
**Chomadoux**
**F-63750 Messeix(FR)**

(74) Mandataire: **Ricalens, François et al**
**RHONE-POULENC CHIMIE Service Brevets**
**Chimie 25, quai Paul Doumer**
**F-92408 Courbevoie(FR)**

(54) **Procédé de carbonylation de l'acide acrylique.**

(57) La présente invention concerne un procédé de préparation d'acide méthylmalonique par carbonylation par le monoxyde de carbone de l'acide acrylique, en solution basique et en présence d'un dérivé carbonylé du fer.

L'acide méthylmalonique sert notamment pour la préparation de la ptérosine C ou dans la formulation de revêtements spéciaux.

EP 0 421 853 A1

EP 0 421 853 A1

## PROCEDE DE CARBONYLATION DE L'ACIDE ACRYLIQUE

La présente invention concerne un procédé de carbonylation catalytique de l'acide acrylique en acide méthylmalonique.

L'acide méthylmalonique sert notamment pour la préparation de la ptérosine C (CAN. JOURNAL CHEM. 1984, 62, 1945) ou dans la formulation de revêtements spéciaux destinés à la détection d'élévations de température (US-A-3 995 489).

Certains de ses sels métalliques présentent des propriétés fongicides (DE-A-954 462).

L'invention consiste plus précisément en un procédé de préparation d'acide méthylmalonique caractérisé en ce que l'on fait réagir l'acide acrylique en solution basique avec le monoxyde de carbone et l'eau en présence d'une quantité efficace de dérivé carbonylé du fer ou de composé susceptible de former un dérivé carbonylé du fer dans le milieu réactionnel.

Comme dérivés carbonylés du fer, on peut citer le fer pentacarbonyle, $Fe_2(CO)_9$, les entités de formule $M^{n+}[HFe(CO)_4]_n^-$ dans laquelle M représente un métal alcalin ou alcalino-terreux et n représente 1 ou 2.

Le dérivé carbonylé du fer est de préférence le fer pentacarbonyle.

La solution basique dans laquelle se déroule la réaction de carbonylation est constituée par une solution au moins partiellement aqueuse d'un hydroxyde de métal alcalin ou de métal alcalino-terreux.

A titre d'exemples de tels hydroxydes, on peut citer l'hydroxyde de sodium, l'hydroxyde de potassium, l'hydroxyde de lithium, l'hydroxyde de calcium, l'hydroxyde de baryum, l'hydroxyde de strontium, l'hydroxyde de magnésium.

L'hydroxyde de calcium qui permet le maintien du pH du milieu réactionnel entre 12 et 13 est tout particulièrement favorable.

La quantité d'hydroxyde dans le milieu réactionnel peut varier largement. Généralement elle représente de 0,1 mole à 5 moles d'hydroxyde pour 1 litre de solvant et de préférence de 0,2 mole/litre à 2 moles/litre.

Le solvant utilisé dans le présent procédé peut être l'eau seule.

On peut également mettre en oeuvre un mélange eau/alcool secondaire ou tertiaire.

Parmi les alcools secondaires ou tertiaires, on peut citer notamment le propanol-2, le méthyl-2 propanol-2 (alcool tertiobutylique), le butanol-2.

Le propanol-2 convient particulièrement bien.

Lorsque le solvant est un mélange eau/alcool secondaire ou tertiaire, le rapport volumique alcool secondaire ou tertiaire/mélange eau-alcool est généralement de 10 % à 60 % et de préférence de 20 % à 50 %.

La température à laquelle est réalisée le procédé de l'invention est habituellement égale ou supérieure à 50°C.

Il n'est pas utile d'opérer à des températures supérieures à 150°C et de préférence la température réactionnelle sera de 60°C à 120°C.

La pression partielle du monoxyde de carbone à la température réactionnelle est généralement de 0,1 MPa à 1 MPa (1 à 10 bars) et de préférence de 0,1 MPa à 0,5 MPa (1 à 5 bars).

La quantité d'acide acrylique dans le milieu réactionnel peut varier très largement. Généralement on met en oeuvre de 0,1 mole à 5 moles d'acide acrylique pour 1 litre de solvant, sans que ces valeurs soient critiques.

La quantité de dérivé carbonylé du fer, et en particulier de fer pentacarbonyle, utilisé comme catalyseur peut également varier dans de larges limites.

Habituellement le rapport molaire dérivé carbonylé du fer/acide acrylique, et plus particulièrement fer pentacarbonyle/acide acrylique, engagé est de 0,1 % à 30 % et de préférence de 0,5 % à 20 %.

Le procédé de l'invention est régiosélectif ; l'acide méthylmalonique est obtenu sans que l'on détecte d'acide succinique.

L'acide méthylmalonique, qui se trouve au moins partiellement sous la forme de son sel de métal alcalin ou alcalino-terreux en fin de carbonylation, est séparé du mélange réactionnel par les méthodes couramment utilisées en chimie.

Les exemples qui suivent illustrent l'invention.

EXEMPLES 1 à 6

Dans un ballon en verre de 250 cm$^3$, muni d'une agitation magnétique, on charge le solvant (eau ou mélange eau/alcool) dont la nature et la quantité sont indiquées dans le tableau 1 ci-après.

2

# EP 0 421 853 A1

On ajoute ensuite la quantité d'hydroxyde de métal alcalin ou alcalino-terreux indiqué dans le tableau ci-après et sous agitation on coule goutte à goutte l'acide acrylique.

Le milieu réactionnel est dégazé par barbotage d'argon pendant 30 minutes, puis l'atmosphère d'argon est purgée par un léger courant de monoxyde de carbone.

Le ballon réactionnel est alors relié à un système fermé comportant une burette graduée remplie de monoxyde de carbone à la pression atmosphérique (0,1 MPa).

Le fer pentacarbonyle est alors additionné dans le ballon à l'aide d'une seringue à travers un capuchon à jupe rabattable et le ballon réactionnel est plongé dans un bain d'huile thermostaté stabilité à la température choisie et solidaire d'une plaque d'agitation magnétique (tournant à 750 tours/minute).

Le déroulement de la réaction est suivi par des prélèvements aliquots.

Le dosage par chromatographie en phase vapeur se fait à l'aide d'un étalon interne (isovalérate de méthyle) après acidification à l'aide de HCl dilué, traitement à 0°C par un excès de diazométhane en solution dans l'éther éthylique et neutralisation de l'excès de diazométhane.

Le tableau 1 ci-après rassemble les caractéristiques de chaque exemple et les résultats obtenus après 48 h de réaction.

## TABLEAU 1

| Exemples | Acide acrylique (en mmol) | Solvants en $cm^3$ | Bases en mmol | $Fe(CO)_5$ (mmol) | T°C | Rdt en AMM % |
|---|---|---|---|---|---|---|
| Ex. 1 | 8,9 | $H_2O$ 50 | KOH (18,3) | 1,4 | 60 | 51 |
| Ex. 2 | 9,0 | $H_2O$ 50 | KOH (18,5) | 1,4 | 70 | 65 |
| Ex. 3 | 8,8 | $H_2O$ 50 | LiOH (18,7) | 1,4 | 60 | 37 |
| Ex. 4 | 9,2 | $H_2O$ 50 | $Ca(OH)_2$ (15,2) | 1,4 | 70 | 66 |
| Ex. 5 | 19,2 | $H_2O$ 40 t.BuOH 10 | $Ca(OH)_2$ (30,2) | 1,4 | 70 | 62 |
| Ex. 6 | 35,0 | $H_2O$ 80 iPrOH 20 | $Ca(OH)_2$ (62) | 0,7 | 70 | 63 |
| Abréviations utilisées dans le tableau 1 | | | | | | |
| - Rdt en AMM % = rendement % en acide méthylmalonique par rapport à l'acide acrylique chargé | | | | | | |
| - t.BuOH = méthyl-2 propanol-2 | | | | | | |
| 9 iPrOH = propanol-2 | | | | | | |

## EXEMPLES 7 et 8

On répète les exemples 1 à 6, mais en fin de réaction (48 h) l'acide méthylmalonique est isolé. Le traitement consiste à refroidir le mélange réactionnel final à -25°C environ, puis à séparer par essorage :
- une partie insoluble A constituée de méthylmalonate de Ca et d'hydroxyde de Ca
- un filtrat B.

Le filtrat est agité à l'air pendant une nuit, puis est filtré pour éliminer les sels de fer.

Le nouveau filtrat est ajouté à la fraction solide A et la solution ainsi obtenue est neutralisée à l'aide d'acide chlorhydrique dilué.

On sature ensuite cette solution par du chlorure de sodium et on l'extrait par 5 x 200 $cm^3$ d'éther éthylique.

Après séchage de la solution éthérée sur du sulfate de Mg et évaporation sous pression réduite du solvant, de l'acide acrylique n'ayant pas réagi et des tracest d'acide propionique formé, on recueille un

3

solide qui est pesé.

La pureté de l'acide méthylmalonique est confirmée par RMN du proton (60 MHz) en solution dans l'eau lourde, en présence de chlorhydrate de pyridinium comme étalon interne.

Le tableau 2 ci-après rassemble les caractéristiques des 2 exemples et les résultats obtenus.

TABLEAU 2

| Exemples | Acide acrylique (en mmol) | Solvants en cm$^3$ | Ca(OH)$_4$ en mmol | Fe(CO)$_5$ (mmol) | T° C | Rdt en AMM % |
|---|---|---|---|---|---|---|
| Ex. 7 | 9,1 | H$_2$O 40 | 23,6 | 1,4 | 70 | 80 |
| | | t.BuOH 10 | | | | |
| Ex. 8 | 35,3 | H$_2$O 80 | 56,8 | 0,7 | 70 | 43 |
| | | t.BuOH 20 | | | | |
| Les abréviations utilisées sont les mêmes que pour le tableau 1 | | | | | | |

## Revendications

1. Procédé de préparation d'acide méthylmalonique caractérisé en ce que l'on fait réagir l'acide acrylique en solution basique avec le monoxyde de carbone et l'eau en présence d'une quantité efficace de de dérivé carbonylé du fer ou de composé susceptible de former un dérivé carbonylé du fer dans le milieu réactionnel.

2. Procédé selon la revendication 1, caractérisé en ce que l'on utilise comme dérivés carbonylés du fer le fer pentacarbonyle, Fe$_2$(CO)$_9$, les entités de formule M$^{n+}$[HFe(CO)$_4$]$_n^-$ dans laquelle M représente un métal alcalin ou alcalino-terreux et n représente 1 ou 2.

3. Procédé selon la revendication 1, caractérisé en ce que l'on utilise comme dérivé carbonylé du fer le fer pentacarbonyle.

4. Procédé selon l'une des revendications 1 à 3, caractérisé en ce que la solution basique est une solution au moins partiellement aqueuse d'un hydroxyde de métal alcalin ou de métal alcalino-terreux.

5. Procédé selon la revendication 4, caractérisé en ce que l'hydroxyde est choisi parmi l'hydroxyde de sodium, l'hydroxyde de potassium, l'hydroxyde de lithium, l'hydroxyde de calcium, l'hydroxyde de baryum, l'hydroxyde de strontium, l'hydroxyde de magnésium et est de préférence l'hydroxyde de calcium.

6. Procédé selon l'une des revendications 4 ou 5, caractérisé en ce que la quantité d'hydroxyde dans le milieu réactionnel représente de 0,1 mole à 5 moles pour 1 litre de solvant et de préférence de 0,2 mole à 2 moles/litre de solvant.

7. Procédé selon l'une des revendications 1 à 6, caractérisé en ce que le solvant utilisé est l'eau.

8. Procédé selon l'une des revendications 1 à 6, caractérisé en ce que le solvant utilisé est un mélange eau/alcool secondaire ou tertiaire dans lequel ledit alcool secondaire ou tertiaire représente de 10 % à 60 % et de préférence de 20 % à 50 % en volume par volume.

9. Procédé selon l'une des revendications 1 à 8, caractérisé en ce que la température est égale ou supérieure à 50° C et est de préférence de 60° C à 120° C.

10. Procédé selon l'une des revendications 1 à 9, caractérisé en ce que l'on opère sous une pression partielle d'oxyde de carbone de 0,1 MPa à 1 MPa et de préférence de 0,1 MPa à 0,5 Mpa.

11. Procédé selon l'une des revendications 1 à 10, caractérisé en ce que l'on met en oeuvre de 0,1 mole à 5 moles d'acide acrylique par litre de solvant.

12. Procédé selon l'une des revendications 1 à 11, caractérisé en ce que le rapport molaire fer pentacarbonyle/acide acrylique engagé est de 0,1 % à 30 % et de préférence de 0,5 % à 20 %.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | DE-C-1006849 (BASF)<br>* revendication 1 *<br>--- | 1 | C07C55/02<br>C07C51/14 |
| A | DE-C-765969 (I.G. FARBEN)<br>* page 2, ligne 41 *<br>--- | 1 | |
| A | US-A-3661949 (FENTON)<br>* colonne 1, lignes 57 - 61 *<br>--- | 1 | |
| A | DE-C-1133359 (LONZA)<br>* page 1, colonne 1, ligne 50 *<br>--- | 1 | |
| A | US-A-2604490 (REPPE)<br>* colonne 2, ligne 3; revendication 1 *<br>--- | 1 | |
| A | US-A-3646131 (IKARASI)<br>* colonnes 7-8 , exemple 8 *<br>--- | 1 | |
| A | CHEMICAL ABSTRACTS, vol. 88, no. 7, 13 février 78<br>Columbus, Ohio, USA<br>& JP-A-52-106811 (MITSUI TOATSU):<br>ref. no. 50294M<br>* abrégé *<br>----- | 1 | **DOMAINES TECHNIQUES RECHERCHES (Int. Cl.5)**<br><br>C07C |

Le présent rapport a été établi pour toutes les revendications

| Lieu de la recherche | Date d'achèvement de la recherche | Examinateur |
|---|---|---|
| BERLIN | 10 JANVIER 1991 | PROBERT,C |